## Europäisches Patentamt

## European Patent Office

## Office européen des brevets

(11) Veröffentlichungsnummer: **0 080 023**
**B1**

(12) **EUROPÄISCHE PATENTSCHRIFT**

(45) Veröffentlichungstag der Patentschrift:
02.01.85

(51) Int. Cl.³: **C 07 C 69/54, C 07 C 67/08**

(21) Anmeldenummer: **82107078.6**

(22) Anmeldetag: **05.08.82**

(54) **Verfahren zum kontinuierlichen Verestern von Methacrylsäure.**

(30) Priorität: **21.11.81 DE 3146191**

(43) Veröffentlichungstag der Anmeldung:
**01.06.83 Patentblatt 83/22**

(45) Bekanntmachung des Hinweises auf die Patenterteilung:
**02.01.85 Patentblatt 85/1**

(84) Benannte Vertragsstaaten:
**BE DE FR GB IT NL**

(56) Entgegenhaltungen:
**DE - A - 2 913 218**
**DE - B - 1 288 596**
**DE - C - 1 693 174**
**FR - A - 1 211 946**

(73) Patentinhaber: **Röhm GmbH, Kirschenallee**
**Postfach 4242, D-6100 Darmstadt 1 (DE)**

(72) Erfinder: **Gänzler, Wolfgang, Dr. Dipl.-Chem., Wixhäuser**
**Strasse 11, D-6100 Darmstadt-Arheilgen (DE)**
Erfinder: **Siegert, Hermann-Josef, Dr. Dipl.-Chem.,**
**Inselstrasse 21, D-6100 Darmstadt (DE)**
Erfinder: **Hohage, Heinz-Jürgen, Dr. Dipl.-Chem., In der**
**Wildnis 3, D-6109 Mühltal (DE)**
Erfinder: **Schröder, Günter, Dr. Dipl.-Chem., Leipziger**
**Strasse 7, D-6105 Ober-Ramstadt (DE)**

**Beschreibung**

Die Erfindung betrifft die Herstellung niederer Ester der Methacrylsäure, insbesondere Methacrylsäuremethylester (MMA), aus einer wäßrigen Methacrylsäurelösung, die weitere Bestandteile, wie Aceton und Essigsäure, enthalten kann. Insbesondere betrifft die Erfindung einen Teil eines Herstellungsverfahrens für niedere Methacrylsäureester, bei dem eine wäßrige Methacrylsäurelösung durch Oxydation bzw. Dehydrierung von Isobutylen, Methacrolein oder vorzugsweise von Isobuttersäure gewonnen und anschließend verestert wird.

Es gibt zahlreiche Beschreibungen derartiger Veresterungsverfahren, die jedoch in der Praxis bisher kaum Anwendung gefunden haben. In der Regel sehen sie zunächst die Extraktion der Methacrylsäure aus der wäßrigen Lösung mit Hilfe eines organischen Lösungsmittels, wie Benzol oder Hexan, vor. Nach destillativer Zerlegung des Extraktes wird die im wesentlichen wasserfreie Methacrylsäure in üblicher Weise verestert.

Als Beispiel sei die DE-A 2 907 602 genannt, wo ein Gemisch aus Xylol und MMA als Extraktionsmittel vorgeschlagen wird. Die Methacrylsäure kann auch in dem Extrakt mit einem Alkanol verestert und der Ester durch Zerlegung des umgesetzten Extraktes gewonnen werden.

Die Einführung eines organischen Lösungsmittels als Extraktionsmittel verursacht zusätzliche Trennprobleme und macht eine Reihe von Trennschritten notwendig, die das Verfahren verteuern.

Aus der US-A 3 663 375 ist es bekannt, wäßrige Methacrylsäure mit soviel anorganischen Salzen, wie Natriumsulfat, zu versetzen, daß sich eine wasserarme Methacrylsäureschicht von der Salzlösung abtrennt. Dieses Verfahren ist jedoch wegen der erforderlichen großen Salzmengen wenig wirtschaftlich.

Ein ähnlicher Weg wird beim Verfahren der US-A 3 821 286 beschritten, wo Schwefelsäure als ein Zusatz verwendet wird, der eine Auftrennung der wäßrigen Methacrylsäurelösung in eine wasserarme organische Schicht und wäßrige Schwefelsäure bewirkt. Die letztere wird durch Erhitzen und Verdampfen von Wasser aufkonzentriert und als Veresterungskatalysator für die zuvor abgeschiedene Methacrylsäureschicht eingesetzt. Das Verfahren sieht zwei Phasentrennungsschritte vor, die in der Praxis langsam und unvollständig ablaufen.

Beim Verfahren der DE-A 2 035 228 wird wäßrige Methacrylsäure unmittelbar durch Zusatz von Schwefelsäure und Methanol verestert. Aus dem Veresterungsgemisch wird MMA durch Extraktion mit organischen Lösungsmitteln gewonnen. Der Extrakt muß destillativ zerlegt werden.

Bei einem ähnlichen Verfahren gemäß DE-A 2 832 202 wird ein mineralsäurefreies Veresterungsgemisch aus MMA, Wasser, Methanol udn Methacrylsäure durch fraktionierte Destillation in ein Destillat aus MMA, Methanol und Wasser, von dem durch Schichttrennung der Hauptteil des Wassers und Methanols abgetrennt wird, und ein Sumpfprodukt aus überwiegenden Mengen Methacrylsäure zerlegt. Das Sumpfprodukt wird in die Veresterungsanlage zurückgeleitet.

Schließlich ist es aus der DE-A 2 509 729 bekannt, wäßrige Methacrylsäure in der Dampfphase an einem Wolframoxidkatalysator zu verestern. Die Veresterung bleibt jedoch unvollständig, so daß Methacrylsäure aus dem Veresterungsgemisch zurückgewonnen werden muß.

Aus dem DE-C 1 693 174 ist die kontinuierliche Veresterung von wasserfreier Methacrylsäure mit niederen Alkoholen im Sumpf einer Destillationsanlage in Gegenwart von 8–30% (berechnet als $SO_3$, bezogen auf Methacrylsäure) an Schwefelsäure oder einer organischen Sulfonsäure bekannt. Bei diesem Verfahren wird der gebildete Ester zusammen mit dem Reaktionswasser laufend abdestilliert, so daß im Sumpf ein Veresterungsgemisch von dauernd gleichbleibender Zusammensetzung vorliegt. Das Destillat wird geschichtet und ein Teil der Esterschicht als Rücklauf in die Destillationsanlage geleitet. Die Durchführung dieses Verfahrens mit wäßriger Methacrylsäure ist nicht bekannt.

Wäßrige Methacrylsäure mit einem Wassergehalt von 5 bis 60 Gew.-% soll ohne Anwendung zusätzlicher organischer Hilfsflüssigkeiten kontinuierlich in einer geringen Zahl von Umsetzungs- und Aufarbeitungsvorrichtungen mit hoher Ausbeute in einen niederen Alkylester übergeführt werden. Die Aufgabe wird durch das Verfahren gemäß Patentanspruch gelöst.

Das erfindungsgemäße Verfahren eignet sich zur Verarbeitung wäßriger Methacrylsäurelösungen mit einem Wassergehalt zwischen 5 und 60 Gew.-%. Bei Wassergehalten über 40, insbesondere über 60 Gew.-% nimmt der Destillationsaufwand für die Abtrennung des Wassers aus dem Veresterungsgemisch in Form eines $MMA/H_2O$-Azeotrops so stark zu, daß die bekannten Extraktionsverfahren wirtschaftlicher sind. Bei einem Wassergehalt unter 5% hat das Verfahren der Erfindung keine Vorzüge vor anderen bekannten Veresterungsverfahren. Vorzugsweise enthält die eingesetzte Methacrylsäure 10 bis 40 Gew.-% Wasser.

Das Verfahren der Erfindung eignet sich auch zur Aufarbeitung wäßriger Methacrylsäurelösungen, die noch weitere, unter den Veresterungsbedingungen flüchtige Bestandteile, wie Essigsäure oder Aceton, enthalten. Der Gehalt an Methacrylsäure soll nicht unter 35% liegen. Es ist überraschend, daß Aceton, das unter Einfluß von Säuren zur Verharzung neigt, nicht zur Bildung von Rückständen in dem Veresterungsgemisch führt.

Der Gehalt an schwerflüchtigen Bestandteilen, z. B. Polymerisationsinhibitoren, in der eingesetzten wäßrigen Methacrylsäure soll möglichst niedrig, im allgemeinen nicht über 1 Gew.-% liegen. Die schwerflüchtigen Bestandteile sammeln sich in dem Veresterungsgemisch an und können durch laufenden Abzug eines Teils der Sumpfphase entfernt werden.

Das Verfahren der Erfindung eignet sich besonders zur Weiterverarbeitung von wäßrigen Methacrylsäurelösungen, die durch Oxydation bzw. Dehydrierung von Isobutylen, Isobutanol, Methacrolein oder insbesondere Isobuttersäure erzeugt worden sind. Sofern die Dehydrierungsprodukte dabei in Wasser aufgenommen werden, wird die Obergrenze von 60% Wasser oft überschritten. Vorteilhafter ist die Kondensation der gasförmigen Oxydations- bzw- Dehydrierungsprodukte durch Abkühlung, weil dann eine Methacrylsäurelösung mit weniger als 60% Wasser gewonnen werden kann. Besonders vorteilhaft ist die Aufarbeitung von Dehydrierungsgemischen, die ohne Wasserdampfzusatz erzeugt worden sind. Ihr Wassergehalt kann beispielsweise zwischen 10 und 40 Gew.-% liegen.

Durch den Verzicht auf Extraktionsmittel entfallen alle Trennprozesse, die sonst mit Rücksicht auf die Reinheit des Endprodukts oder auf Rückstände in Abgasen oder Abwässern unumgänglich sind.

Obwohl befürchtet werden mußte, daß durch das Einbringen von erheblichen Mengen von Wasser in ein Verfahren das sonst mit wasserfreier Methacrylsäure durchgeführt wurde, das Veresterungsgleichgewicht weit auf die Seite der Säure verschoben und eine vollständige Veresterung der zugeführten Methacrylsäure unterbunden würde, wurde überraschenderweise gefunden, daß die Veresterung mit hoher Geschwindigkeit unter vollständigem Umsatz der eingeführten Methacrylsäure durchgeführt werden kann.

Dank der vollständigen Umsetzung der eingesetzten Methacrylsäure zum Ester braucht keine Methacrylsäure zurückgewonnen zu werden. Aus dem Veresterungsgemisch entweichen lediglich dampfförmige Bestandteile, die auf einfache Weise destillativ getrennt werden können. Daher kommt das Verfahren mit einem Minimum an Trenn- und Aufarbeitungsvorrichtungen aus. Es entstehen weder Abwasser- noch Abgasprobleme.

Das Veresterungsgemisch besteht überwiegend aus einem Gemisch von Methacrylsäure und der als Katalysator dienenden Schwefelsäure oder Sulfonsäure. Schwefelsäure ist bevorzugt. Als organische Sulfonsäure kann z. B. Benzolsulfonsäure, Toluolsulfonsäure oder Methansulfonsäure eingesetzt werden. Verwendbar sind alle unter den Reaktionsbedingungen flüssigen, nicht flüchtigen und beständigen Sulfonsäuren. Der Anteil dieses Katalysators in dem Veresterungsgemisch wird so bemessen, daß der in der Schwefel- oder Sulfonsäure enthaltene $SO_3$-Bestandteil in einer Konzentration von 50 bis 500 g/l vorliegt. Im laufenden Betrieb kann ein Teil der eingesetzten Schwefelsäure in Monoalkylschwefelsäure übergehen.

Die Menge des im stationären Zustand vorliegenden Veresterungsgemisches kann 5 bis 200 Gew.-%, bezogen auf die pro Stunde zulaufende wäßrige Methacrylsäure betragen.

Gleichzeitig mit der wäßrigen Methacrylsäure wird dem Gemisch ein niederes Alkanol in wenigstens äquivalenter Menge, gegebenenfalls in einem stöchiometrischen Überschuß bis zum doppelten des Methacrylsäurezulaufs, kontinuierlich zugesetzt. Das bevorzugte Mol-Verhältnis von Methacrylsäure zu Alkanol liegt bei 1 zu 1,2 bis 1,6. Verwendbar sind niedere Alkanole mit höchstens 4 C-Atomen. Methanol ist bevorzugt. Weitere wichtige Alkanole sind Äthanol, Isopropanol, n-Propanol, Isobutanol und n-Butanol. Das eingesetzte Alkanol kann eine kleine Menge Wasser enthalten. Jedoch soll der Wassergehalt des Alkanols und der wäßrigen Methacrylsäure zusammengenommen nicht über 60 Gew.-%, bezogen auf die Gewichtssumme aus dem Wasser und der Methacrylsäure betragen.

Das Veresterungsgemisch wird vorzugsweise bei vermindertem Druck, bei Tempraturen zwischen 70 und 170°C am Sieden gehalten. Dazu sind Drucke von 60 bis 1000 mbar erforderlich. Das Veresterungsgemisch bildet den Sumpf einer Destillationsanlage.

In dem Fall, daß sich in dem Veresterungsgemisch nichtflüchtige Rückstände ansammeln, kann kontinuierlich oder von Zeit zu Zeit ein Teil des Gemisches abgezogen und verworfen oder auf geeignete Weise gereinigt werden. Wenn feste, ungelöste Rückstände entstehen, können sie durch Filtration beseitigt und das Filtrat in den Sumpf zurückgeführt werden.

Der Sumpf wird mittels eines Heizmantels oder einer Heizschlange erhitzt und in solchem Maße am Sieden gehalten, daß die Menge des Gemisches dauernd gleichbleibt. Zweckmäßig ist der Sumpf als Umlaufverdampfer ausgebildet, um eine gute Durchmischung des Veresterungsgemisches ohne mechanische Rührung zu gewährleisten.

Das Destillat, das aus dem Sumpf entweicht, wird vorzugsweise über eine Destillationskolonne abgenommen. Das Destillat besteht in der Regel aus einem Gemisch aus dem Ester-Wasser-Azeotrop und dem Alkanol. Das MMA-Wasser-Azeotrop hat bei Normaldruck die Zusammensetzung 86% MMA und 14% Wasser. Nach dem Kondensieren trennt sich das Destillat in eine obere Esterschicht und eine untere Wasserschicht, die in einem Absitzgefäß voneinander getrennt werden. Ein Teil, in der Regel der überwiegende Teil, der Esterschicht wird als Rücklauf wieder in die Kolonne geleitet, ein Teil davon zweckmäßig in den mittleren bis unteren Abschnitt der Kolonne, wodurch die Trennwirkung verbessert wird. Das Rücklaufverhältnis richtet sich nach der Menge des zuströmenden und des bei der Veresterung entstehenden Wassers. Als Faustregel kann gelten, daß für 1 Teil Wasser 6 bis 7 Teile des Esters abdestilliert werden müssen.

Die Esterschicht kann neben dem Hauptprodukt noch Hoch- und Niedersieder enthalten, die in bekannter Weise durch mehrere Destillationsstufen voneinander getrennt werden können. Häufig enthält die eingesetzte wäßrige Methacrylsäure eine kleine Menge Isobuttersäure. Diese wird gleichzeitig mit der Methacrylsäure verestert udn kann aus dem entsprechenden Gemisch der Ester durch Destillation abgetrennt werden. Dazu ist eine hohe Bodenzahl der Destillationskolonne erforderlich, jedoch ist die Destillationsarbeit dennoch ziemlich gering, weil der Isobuttersäureester im allgemeinen in geringer Menge vorliegt und als Kopfprodukt abgenommen wird. Es entsteht also nur eine geringe Destillatmenge, die darüber hinaus nicht vollständig von dem Methacrylsäureester befreit zu werden braucht.

Die wäßrige Phase des Kondensats enthält neben Wasser die Hauptmenge des nicht eingesetzten Alkanols, z. B. Methanol, sowie, entsprechend der Löslichkeit, Nebenbestandteile, wie z. B. Aceton. Das Alkanol kann fraktioniert abdestilliert und in das Veresterungsgemisch zurückgeleitet werden.

Zur Verhinderung unerwünschter Polymerisation kann — vorzugsweise auf den Kopf der Destillationskolonne — ein Polymerisationsinhibitor zugefügt werden. Bevorzugt sind schwerflüchtige Inhibitoren, wie Hydrochinon oder Hydrochinonmonomethyläther. Sie gelangen allmählich in den Destillationssumpf und werden bei dessen Aufarbeitung entfernt. Man kann auch kleine Mengen Sauerstoff durch die Anlage leiten.

## Beispiele

### A. Beschreibung der Laborapparatur

Die Sumpfblase ist als Umlaufverdampfer (Reaktionsvolumen: 130 ml) ausgebildet und mit Zuführungen für wasserhaltige Rohmethacrylsäure, Alkanol und eine geringe Menge Luft versehen. Die Heizung erfolgt über einen Ölumlauf im Heizmantel. Aufgesetzt ist eine Destillationskolonne (Länge 90 cm, Innendurchmesser 2,5 cm), die mit einer Füllkörperschüttung gefüllt ist. Im unteren Drittel ist eine Zuführung für vorgewärmten oder verdampften »Rückester« eingebaut, der aus der Esterphase des Destillats oder aus dem Reindestillat der Esterphase abgezweigt wird. Der Kolonnenkopf ist mit einem Rücklaufregler ausgestattet, um einen direkten Rücklauf auf die Kolonne zu gewährleisten.

Im Totalkondensator wird die gesamte aus der Kolonne austretende Dampfphase kondensiert. Alle Komponenten teilen sich entsprechend ihrer Löslichkeit und den Verteilungskoeffizienten in zwei Phasen auf. Die obere organische, vorwiegend den Ester enthaltende Phase wird von der unteren wäßrigen, vorwiegend das ausgeschleppte Wasser enthaltenden Phase getrennt.

Die Apparatur kann bei vermindertem oder Normaldruck betrieben werden.

### B. Katalysator

Als Anfangskatalysator wird konzentrierte Schwefelsäure eingesetzt. Die jeweilige Menge ist in der nachfolgenden Tabelle angegeben.

Mit einem Katalysatoreinsatz wurde nach Laufzeiten von 175 Stunden keine merkliche Einbuße an Aktivität festgestellt.

### C. Durchführung

Im Sumpf der Veresterungsapparatur wird der Anfangskatalysator (z. B. konzentrierte Schwefelsäure), zu veresterndes Gemisch aus Rohmethacrylsäure und Alkohol sowie ein Inhibitor vorgelegt. Die Apparatur wird dann auf Betriebsbedingungen von Druck und Temperatur gebracht.

Dann werden kontinuierlich Rohmethacrylsäure und Alkohol im gewünschten Mischungsverhältnis mit einer solchen Geschwindigkeit eindosiert, daß das Sumpfvolumen konstant bleibt.

Gleichzeitig wird Methacrylester bzw. Rohester, der nach der Phasentrennung aus der organischen Destillatphase entnommen worden ist, als Dampf oder vorgewärmte Flüssigkeit im unteren Teil der Destillationskolonne eingespeist. Dieser Mengenstrom muß so groß sein, daß im Destillat keine Carbonsäure nachgewiesen werden kann.

Im stationären Zustand wird ein Teil des als organische Phase anfallenden Rohesters in die Destillationskolonne zurückgeführt.

Bei den Beispielen 1—4 besteht die eingesetzte Rohmethacrylsäure aus 30 Gew.-% Wasser und 70 Gew.-% Methacrylsäure. In den Beispielen 5—9 wird ein Gemisch aus 74,1% Methacrylsäure, 1,0% Isobuttersäure, 21,7% Wasser, 2,1% Essigsäure und 1,1% Aceton eingesetzt.

In der folgenden Tabelle sind für eine Reihe von stationären Betriebszuständen die Bedingungen und Mengenströme zusammengestellt.

| Beispiel Nr. | konz. $H_2SO_4$ (g) | Druck (mbar) | Dosierung (g/h) Rohsäure | $H_2O$-Gehalt d. Meth-acrylsäure (%) | Methanol | Rückester Menge | MMA-Gehalt | Molverh. Methanol zu Gesamtsäure | Methylmeth-acrylat (g/h) | Ausbeute % d. Th. |
|---|---|---|---|---|---|---|---|---|---|---|
| 1 | 25 | 329 | 176,5 | 30 | 73,5 | 404 | 95% | 1,6 : 1 | 138,5 | 96,5 |
| 2 | 50 | 329 | 221 | 30 | 69 | 465 | 100% | 1,2 : 1 | 179 | 99,5 |
| 3 | 50 | 329 | 165 | 30 | 51,6 | 358 | 95% | 1,2 : 1 | 132 | 98 |
| 4 | 50 | 329 | 195 | 30 | 66 | 372 | 95% | 1,3 : 1 | 156 | 98 |
| 5 | 50 | 329 | 248 | 21,7 | 86 | 497 | 93% | 1,20 : 1 | 197 | 92 |
| 6 | 50 | 329 | 340 | 21,7 | 124 | 572 | 95% | 1,25 : 1 | 288 | 98,5 |
| 7 | 50 | 329 | 325 | 21,7 | 117 | 567 | 95% | 1,225 : 1 | 276 | 98,5 |
| 8 | 50 | 658 | 344 | 21,7 | 122,5 | 475 | 94% | 1,225 : 1 | 294 | 98,5 |
| 9 | 25 | 329 | 228 | 21,7 | 81 | 381 | 95% | 1,225 : 1 | 194 | 98 |

## Patentanspruch

Verfahren zum kontinuierlichen Verestern von Methacrylsäure mit einem niederen Alkanol durch Einleiten der Methacrylsäure und einer wenigstens äquivalenten Menge des Alkanols in ein siedendes Veresterungsgemisch mit einer Temperatur von 70 bis 170°C, das Schwefelsäure oder eine organische Sulfonsäure in einer Menge entsprechend 50 bis 500 g $SO_3$/l enthält, Abdestillieren eines Methacryl-säureester-Wasser-Azeotrops, Schichtung des kondensierten Destillats in eine obere Esterschicht und eine untere Alkanol-Wasser-Schicht, Rückführung eines Teils der Esterschicht als Rücklauf in die Destillation des Azeotrops, dadurch gekennzeichnet, daß wäßrige Methacrylsäure mit einem Wassergehalt von 5 bis 60 Gew.-% in das Veresterungsgemisch eingeleitet und das damit eingebrachte Wasser sowie das bei der Veresterung gebildet Wasser laufend in Form des genannten Azeotrops abdestilliert wird.

## Claim

Process for continuously esterifying methacrylic acid with a lower alkanol by introducing methacrylic acid and an at least equivalent quantity of the alkanol into a boiling esterification mixture at a temperature of from 70 to 170°C which contains sulphuric acid or an organic sulphonic acid in a quantity corresponding to 50 to 500 g $SO_3$/l, distilling off a methacrylate/water azeotrope, stratifying the condensed distillate into an upper ester layer and a lower alkanol/water layer, recycling part of the ester layer as returned material into the distillation of the azeotrope, characterized in that aqueous methacrylic acid with a water content of from 5 to 60% by weight is introduced into the esterification mixture and the water thus added together with the water formed during the esterification is continuously distilled off in the form of the above mentioned azeotrope.

## Revendication

Procédé d'estérification en continu d'acide méthacrylique avec un alcanol inférieur, par introduction de l'acide méthacrylique et d'une quantité au moins équivalente de l'alcanol dans un mélange d'estérification bouillant qui est à une température de 70 à 170°C et qui contient de l'acide sulfurique ou un acide sulfonique organique dans une proportion correspondant à 50—500 g de $SO_3$/litre, élimination par destillation d'un azéotrope d'ester d'acide méthacrylique et d'eau, séparation de phases du distillat condensé en une couche supérieure d'ester et une couche inférieure d'alcanol-eau et recyclage d'une partie de la phase ester comme reflux dans la distillation de l'azéotrope, caractérisé en ce qu'on introduit dans le mélange d'estérification un acide méthacrylique aqueux ayanat une teneur en eau de 5 à 60% en poids et en ce que l'eau ainsi introduite, ainsi que l'eau formée au cours de l'estérification sont chassées en permanence par distillation sous la forme dudit azéotrope.